# EUROPEAN PATENT APPLICATION

(11) **EP 2 314 579 A1**
(43) Date of publication of application: **27.04.2011**
(21) Application number: 09173728.8
(22) Date of filing: 22.10.2009
(51) Int. Cl.: C07D 265/16, C08G 73/02

(54) **Fast-curing benzoxazine compounds**

(71) Applicant: Henkel AG & Co. KGaA, 40589 Düsseldorf (DE)
(72) Inventor: Kudoh, Ryoichi, Otsu Shiga 520-0002 (JP); Sudo, Atsushi, Nerima-ku Tokyo 177-0044 (JP); Endo, Takeshi, Yokohama 220-0006 (JP)

(57) **Abstract**

The present invention relates to a specific benzoxazine compound A of formula (I), 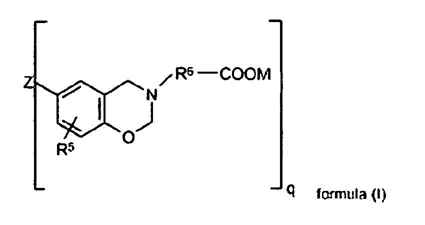 wherein q is an integer from 1 to 4, M is hydrogen, a monovalent cation, or an equivalent of a polyvalent cation, Z is selected from the group consisting of a direct bond (when q is 2), hydrogen (when q is 1), alkyl (when q is 1), alkylene (when q is 2 to 4), carbonyl (when q is 2), oxygen (when q is 2), thiol (when q is 1), sulfur (when q is 2), sulfoxide (when q is 2), and sulfone (when q is 2), R⁶ is a linear or branched divalent alkylene group, comprising 1 to 15 carbon atoms, which may be interrupted by one or more heteroatom(s), selected from oxygen, nitrogen and sulfur, and R⁵ is selected from hydrogen, halogen, alkyl, alkenyl or R⁵ is a divalent residue creating a naphthoxazine residue out of the benzoxazine structure. A further object of the present invention is a polymerizable composition and a cured product obtained from the polymerizable composition, wherein said composition comprises at least one benzoxazine compound A and at least one benzoxazine compound B, which is different from benzoxazine compound A.

## Description

### FIELD OF THE INVENTION

The present invention relates to a specific benzoxazine compound A, which comprises at least one carboxyl group or a corresponding salt thereof. A further object of the present invention is a polymerizable composition and a cured product obtained from the polymerizable composition, wherein said composition comprises at least one benzoxazine compound A and at least one benzoxazine compound B, which is different from benzoxazine compound A.

### DESCRIPTION OF THE PRIOR ART

Normally, benzoxazines are cured at relatively high temperatures. In order to reduce the polymerization temperature of benzoxazines, various curatives/catalysts, like phenols (JP2000-178332A), amines (JP2000-86863A), imidazoles (JP 2000-178332A), and phosphines (JP 2003-82099A) have been reported. US 6,225,440 B1 discloses Lewis acids, such as PCl₅, TiCl₄, AlCl₃ as highly active curative/catalyst for the polymerization of benzoxazines. However, in practical applications, such strong Lewis acids negatively contribute to the final polymerization result and its practical properties. For example deterioration of chemical resistance and physical properties of the cured material may appear. Additionally, Lewis acids, such as PCl₅, TiCl₄, AlCl₃ are highly sensitive to moisture and could cause the formation of volatile, toxic and corrosive impurities.

Alternative curatives/catalysts based on carboxylic acids have also been reported. JP2001-213957A discloses the use of aliphatic and/or aromatic carboxylic acids, such as acetic acid, lactic acid, benzoic acid, and malonic acid as curatives/catalysts for the low-temperature curing of benzoxazines.

In this regard, US 6,376,080 B1 teaches a method of preparing a polybenzoxazine. In said method heterocyclic dicarboxylic acids are used as low-temperature curatives/catalysts.

However, taking into account that additional curatives/catalysts could cause the formation of volatile, toxic and corrosive impurities, it would be desirable to provide new benzoxazine compounds which can be polymerized/cured at relatively low temperatures in short time periods without using additional curatives/catalysts. In this regard, it would also be desirable to provide new benzoxazine-based compositions, which can be polymerized/cured in an environmentally friendly process at relatively low temperatures in short time periods, without using toxic and/or corrosive compounds.

### SUMMARY OF THE INVENTION

The present invention provides a benzoxazine compound of formula (I), 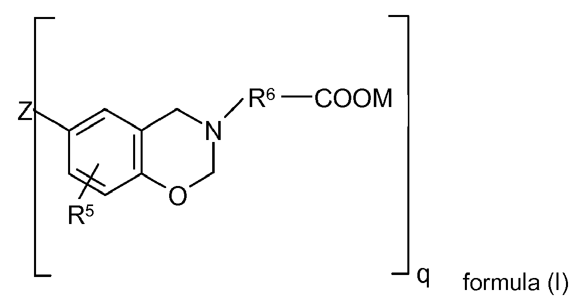 wherein q is an integer from 1 to 4, M is hydrogen, a monovalent cation, or an equivalent of a polyvalent cation, Z is selected from the group consisting of a direct bond (when q is 2), hydrogen (when q is 1), alkyl (when q is 1), alkylene (when q is 2 to 4), carbonyl (when q is 2), oxygen (when q is 2), thiol (when q is 1), sulfur (when q is 2), sulfoxide (when q is 2), and sulfone (when q is 2), R⁶ is a linear or branched divalent alkylene group, comprising 1 to 15 carbon atoms, which may be interrupted by one or more heteroatom(s), selected from oxygen, nitrogen and sulfur and R⁵ is selected from hydrogen, halogen, alkyl, alkenyl or R⁵ is a divalent residue creating a naphthoxazine residue out of the benzoxazine structure.

It is further provided a method of preparing the benzoxazine compound A of the present invention.

In this regard it is another object of the present invention to provide a polymerizable composition, comprising at least one benzoxazine compound A of the present invention and at least one benzoxazine compound B, which is different from benzoxazine compound A.

The polymerizable compositions or the benzoxazine compounds A of the present invention are in particular suitable as coatings, adhesives, sealants and matrices for the preparation of reinforced material such as prepregs and towpregs and/or can be used in injection molding or extrusion.

Therefore it is another object of the invention to provide an adhesive, sealant or coating, comprising the benzoxazine compound A or a polymerizable composition of the present invention and a cured reaction product of the benzoxazine compound A or of the polymerizable composition of the present invention, in particular a cured reaction product containing bundles or layers of fibers. It is further provided a method of preparing such material.

In a further object the invention features a method to increase the polymerization rate of a curable composition at temperatures up to 200°C.

In another object of the present invention the at least one benzoxazine compound A of the present invention is used as a curative/catalyst for curable compositions, comprising at least one benzoxazine compound, which is different from benzoxazine compound A.

### DETAILED DESCRIPTION OF THE INVENTION

The at least one benzoxazine compound A is represented by formula (I), 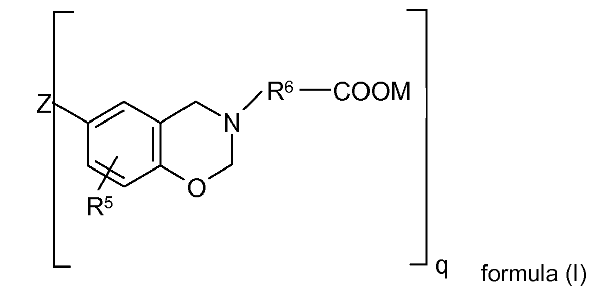 wherein q is an integer from 1 to 4, M is hydrogen, a monovalent cation, or an equivalent of a polyvalent cation, Z is selected from the group consisting of a direct bond (when q is 2), hydrogen (when q is 1), alkyl (when q is 1), alkylene (when q is 2 to 4), carbonyl (when q is 2), oxygen (when q is 2), thiol (when q is 1), sulfur (when q is 2), sulfoxide (when q is 2), and sulfone (when q is 2), R⁶ is a linear or branched divalent alkylene group, comprising 1 to 15 carbon atoms, which may be interrupted by one or more heteroatom(s), selected from oxygen, nitrogen and sulfur and R⁵ is selected from hydrogen, halogen, such as fluorine, chlorine, bromine or iodine, alkyl, alkenyl or R⁵ is a divalent residue creating a naphthoxazine residue out of the benzoxazine structure.

In a specific embodiment of the present invention q is an integer selected from 1 or 2 and/or Z is selected from hydrogen, a direct bond or an alkylene group, comprising 1 to 4 carbon atoms.

In certain embodiments of the present invention R⁵ is selected from the group consisting of hydrogen, methyl, ethyl, n-propyl, iso-propyl, n-butyl, sec-butyl and iso-butyl. Preferably R⁵

The divalent alkylene group R⁶ includes saturated or unsaturated, substituted or unsubstituted divalent alkylene groups and may be interrupted by one more heteroatom(s). The term "interrupted by" means that at least one non-terminal carbon atom of the divalent alkylene group is replaced by a heteroatom selected from the group consisting of *--S--* (sulfur),*--O--* (oxygen), and *--NH--* (nitrogen).

In one embodiment of the present invention the alkylene group R⁶ is not interrupted by one or more heteroatom(s), and contains from 2 to about 8 carbon atoms. Preferably R⁶ can be selected from linear divalent alkylene groups, such as ethylene, propylene, butylene, pentylene, and hexylene.

In another embodiment of the present invention R⁶ is a linear divalent alkylene group, comprising 1 to 4 carbon atoms, preferably 2 to 3 carbon atoms. Inventive benzoxazine compounds A having a divalent alkylene group R⁶ of 1 to 4 carbon atoms are advantageous because said compounds undergo polymerization at very low temperatures even if no additional curative is present to accelerate and/or initiate the polymerization reaction.

In a further embodiment of the present invention, the divalent alkylene group R⁶ is interrupted by oxygen. Preferably R⁶ is a linear divalent alkylene group interrupted by at least one oxygen atom. More preferably R⁶ is a linear divalent alkylene group interrupted by one oxygen heteroatom and said alkylene group comprises from 3 to 6 carbon atoms.

If the divalent alkylene group R⁶ is interrupted by more than one heteroatom, such as oxygen, it is preferred that the shortest atom chain between two heteroatoms contains at least 2 consecutive carbon atoms. Inventive Benzoxazine compounds A having a divalent alkylene group R⁶ which is interrupted by one or more heteroatom(s), such as oxygen are advantageous, because said compounds exhibit a high water-solubility and could therefore be used in water-based applications.

The benzoxazine compound A of the present invention can form salts which are also within the scope of this invention. Normally these salts comprise said benzoxazine compounds A in form of carboxylate anions, wherein said anions are accompanied by an equivalent quantity of cations M that balance the overall charge. In formula (I) M represents a monovalent cation, or an equivalent of a polyvalent cation.

As used herein the term "monovalent cation" refers to positively charged cations with a charge of +1.

As used herein the term "polyvalent cation" refers to positively charged cations with a charge of +2 or greater.

Examples of suitable cations M include: inorganic cations such as alkali metal cations (for example, Li⁺, N⁺, K⁺), alkaline earth metal cations, transition metal cations, and NH₄⁺; organic cations such as, for example, onium cations like primary, secondary, tertiary or quaternary ammonium cations, sulfonium cations, phosphonium cations; and combinations thereof.

Preferred cations M are singly charged (monovalent) and/or can be selected from cations which comprise at least one heteroatom selected from nitrogen, phosphorous or sulfur. In order to limit the concentration of metals in a composition containing the benzoxazine compound A of the present invention, it is preferred to use non-metallic cationic species as cation M.

Suitable non-metallic cations include quaternary ammonium cations, phosphonium cations, or sulfonium cations and/or mixture or combinations thereof.

In preferred embodiments of the present invention each cation M in formula (I) is independently selected from
- quaternary ammonium cations of general formula (II)

   [NR^{a}R^{b}R^{c}R^{d}]⁺ formula (II)
- phosphonium cations of general formula (III), or

   [PR^{a}R^{b}R^{c}R^{d}]⁺ formula (III)
- sulfonium cations of general formula (IV),

   [SR^{a}R^{b}R^{c}]⁺ formula (IV)
wherein the residues R^{a}, R^{b}, R^{c}, and R^{d} (if present) are independently selected from C₁-C₄₀ alkyl groups, C₃-C₄₀ cycloalkyl groups, C₃₋₄₀ alkenyl groups, C₃₋₄₀ alkynyl groups, C₇₋C₄₀ aralkyl groups, C₆-C₄₀ aryl groups or C₇-C₄₀ aralkyl groups.

The term "C₁₋₄₀ alkyl" denotes branched and unbranched alkyl groups with 1 to 40 carbon atoms. Preferred are alkyl groups with 1 to 4 carbon atoms. Examples include: methyl, ethyl, n-propyl, iso-propyl, n-butyl, iso-butyl, sec-butyl, tert-butyl, n-pentyl, iso-pentyl, neo-pentyl or hexyl. The definitions propyl, butyl, pentyl and hexyl include all possible isomeric forms of the groups in question. Thus, for example, propyl includes n-propyl and iso-propyl, butyl includes iso-butyl, sec-butyl and tert-butyl etc. Unless otherwise stated, the alkyl groups may be substituted by one or more groups preferably selected from methyl, ethyl, iso-propyl, tert-butyl, hydroxy, fluorine, chlorine, bromine and iodine.

The term "C₃₋₄₀ cycloalkyl" denotes cyclic alkyl groups with 3 to 40 carbon atoms. Examples include: cylopropyl, cyclobutyl, cyclopentyl or cyclohexyl. Unless otherwise stated, the cyclic alkyl groups may be substituted by one or more groups preferably selected from among methyl, ethyl, iso-propyl, tert-butyl, hydroxy, fluorine, chlorine, bromine and iodine.

The term "C₃₋₄₀ alkenyl" denotes branched and unbranched alkenyl groups with 3 to 40 carbon atoms. Preferred are alkenyl groups with 3 to 5 carbon atoms. Examples include: propenyl, butenyl, pentenyl, or hexenyl. Unless otherwise stated, the definitions propenyl, butenyl, pentenyl and hexenyl include all possible isomeric forms of the groups in question. Thus, for example, propenyl includes 1-propenyl and 2-propenyl, butenyl includes 1-, 2- and 3-butenyl, 1-methyl-1-propenyl, 1-methyl-2-propenyl etc.

The term "C₃₋₄₀-alkynyl" denotes branched and unbranched alkynyl groups with 3 to 40 carbon atoms. Preferred are alkynyl groups with 3 to 5 carbon atoms. Examples include: propynyl, butynyl, pentynyl or hexynyl. Unless otherwise stated, the definitions propynyl, butynyl, pentynyl and hexynyl include all possible isomeric forms of the groups in question. Thus, for example, propynyl includes 1-propynyl and 2-propynyl, butynyl includes 1-, 2- and 3-butynyl, 1-methyl-1-propynyl, 1-methyl-2-propynyl etc.

The term "aryl" denotes aromatic ring systems with 6 to 40 carbon atoms. Examples include: phenyl or naphthyl, the preferred aryl group being phenyl. Unless otherwise stated, the aromatic groups may be substituted by one or more groups preferably selected from among methyl, ethyl, iso-propyl, tert-butyl, hydroxy, fluorine, chlorine, bromine and iodine.

The term "C₇₋₄₀ aralkyl" denotes branched and unbranched alkyl groups with 1 to 30 carbon atoms which are substituted by an aromatic ring system with 6 or 10 carbon atoms. Examples include: benzyl, 1- or 2-phenylethyl. Unless otherwise stated, the aromatic groups may be substituted by one or more groups preferably selected from among methyl, ethyl, iso-propyl, tert-butyl, hydroxy, fluorine, chlorine, bromine and iodine.

Examples of suitable quaternary ammonium cations include tetra-methyl-ammonium, tetraethyl-ammonium, tetra-(n-propyl)-ammonium, tetra-(isopropyl)-ammonium, tetra-cyclopropyl-ammonium, tetra-(n-butyl)-ammonium, tetra-(isobutyl)-ammonium, tetra-(tert-butyl)-ammonium, tetra(sec-butyl)-ammonium, tetra-cyclobutyl-ammonium, tetra-(n-pentyl)-ammonium, tetra-cyclopentyl-ammonium, tetra-(n-hexyl)-ammonium, tetra-cyclohexyl-ammonium, and mixtures thereof.

Examples of suitable phosphonium cations include tetra-methyl-phosphonium, tetra-ethyl-phosphonium, tetra-(n-propyl)-phosphonium, tetra-(isopropyl)-phosphonium, tetra-cyclopropyl-phosphonium, tetra-(n-butyl)-phosphonium, tetra-(isobutyl)-phosphonium, tetra-(tert-butyl)-phosphonium, tetra(sec-butyl)-phosphonium, tetra-cyclobutyl-phosphonium, tetra-(n-pentyl)-phosphonium, tetra-cyclopentyl-phosphonium, tetra-(n-hexyl)-phosphonium, tetra-cyclohexyl-phosphonium, and mixtures thereof.

Examples of suitable sulfonium cations include tri-methyl-sulfonium, tri-ethyl-sulfonium, tri-(n-propyl)-sulfonium, tri-(isopropyl)-sulfonium, tri-cyclopropyl-sulfonium, tri-(n-butyl)-sulfonium, tri-(isobutyl)-sulfonium, tri-(tert-butyl)-sulfonium, tri-(sec-butyl)-sulfonium, tri-cyclobutyl-sulfonium, tri-(n-pentyl)-sulfonium, tri-cyclopentyl-sulfonium, tri(n-hexyl)-sulfonium, tri-cyclohexyl-sulfonium, and mixtures thereof.

Specific examples of benzoxazine compounds A of the present invention include: 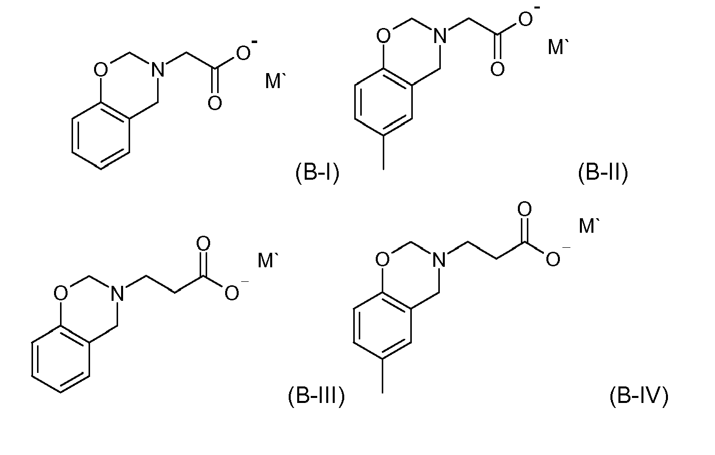 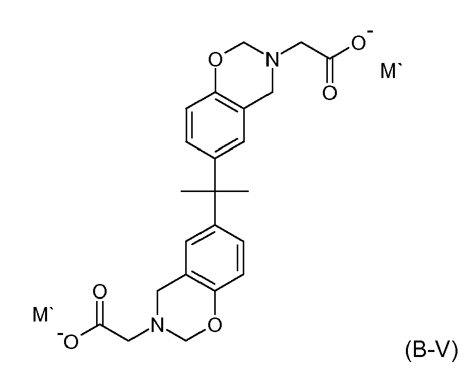 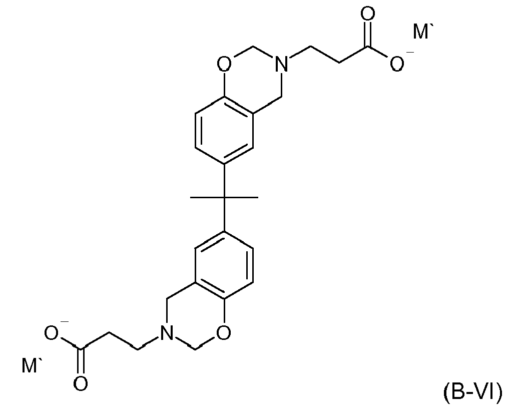 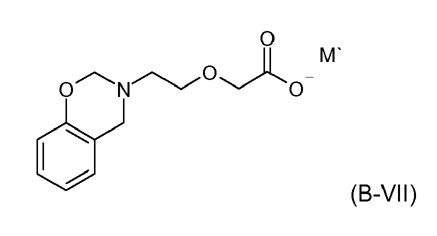 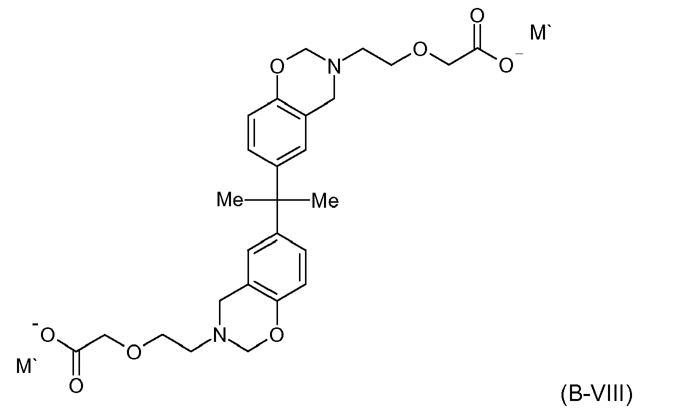 wherein each M' in formula (B-I) to (B-VIIII) is independently selected from [N(n-Bu)₄]⁺ or [P(n-Bu)₄]⁺.

The benzoxazine compound A of the present invention can be prepared according to any method. A preferred method of preparing the benzoxazine compound A comprises the steps of:
(a) providing a reaction mixture, comprising
   i) at least one phenolic compound,
   ii) at least one primary amine of formula (VII),

      H₂N-R⁶-COOM formula (VII),

      wherein M is hydrogen, a monovalent cation or an equivalent of a polyvalent cation, R⁶ is a linear or branched divalent alkylene group, comprising 1 to 15 carbon atoms, which may be interrupted by one or more heteroatom(s), selected from oxygen, nitrogen and sulfur,
   iii) formaldehyde or a reactant releasing formaldehyde, and
   iv) at least one solvent;
(b) heating the reaction mixture under reflux,
(c) optionally removing water from the reaction mixture, and
(d) separating the benzoxazine compound A from the solvent.

The at least one phenolic compound can be selected from monophenols and/or diphenols. The most simple of such diphenols are 1,2-dihydroxy benzene, 1,3-dihydroxy benzene and 1,4-dihydroxy benzene. A diphenol with two phenolic hydroxyl groups attached to different benzene residues is, e.g. biphenyl-4,4'-diol (also known as "4,4'-Biphenol"). Other suitable examples for diphenols are, e.g. Bisphenol A, Bisphenol P, Bisphenol M, Bisphenol F, Bisphenol S, Bisphenol AP, Bisphenol E, 4,4'-oxydiphenol, 4,4'-thiodiphenol, bis(4-hydroxyphenyl)methanone, biphenyl-2,2'-diol, 4,4'-(cyclohexane-1,1-diyl)diphenol or 4,4'-(3,3,5-trimethylcyclohexane-1,1-diyl)diphenol (Bisphenol TMC). Suitable monophenols include phenol, ortho cresol, meta-cresol and para-cresol.

In formula (VVII) the divalent alkylene group R⁶ and the cation M are defined as in the benzoxazine compound of formula (I).

The primary amines of formula (VII) can be obtained by treating an amino acid, like alanine, beta-alanine and/or gamma-alanine with a base, such as tetra-(n-butyl)ammonium hydroxide, and/or tetra-phenyl-phosphonium hydroxide.

In a preferred embodiment of the inventive method the reaction is carried out under inert gas atmosphere and/or
(a) heating the reaction mixture under reflux is carried out for 1 to 10 hours, and/or
(b) removing water from the reaction mixture is carried out by azeotropic distillation.

In a further embodiment of the inventive method the reaction mixture is formed by combining the at least one phenolic compound, the at least one primary amine of formula (VII) and formaldehyde or a reactant releasing formaldehyde at a temperature no warmer than about 30 °C.

In particular, a reaction vessel is kept at a temperature of no warmer than about 100 °C, preferably no warmer than about 70°C and most preferably no warmer than about 30 °C while the reactants are added, preferably under an inert gas atmosphere, such as nitrogen gas. The reaction vessel may be cooled with ice, or any other cooling mechanism. The reactants can be dissolved or dispersed in solvents, such as toluene, ethyl acetate and/or ethanol, preferably before adding to the vessel. Most preferably the reactants are added in small amounts to ensure that the temperature is maintained as desired.

One preferred solvent is ethanol. The combination of solvents is also advantageous in that it allows the separation of water from solvent in a Barrett or Dean-Stark distillation trap to be sharp and allowing nearly all the solvent to be returned to the reaction vessel. However, depending on the solubility of the benzoxazine compound A it can also be preferred to use only toluene as a solvent. Other solvents such as xylene, cyclohexane and chloroform, or water soluble solvents as tetrahydrofuran, dioxane, or propanol can also be used.

In general the reaction mixture is slowly warmed to a temperature at which an exothermic reaction in form of rapid boiling occurs. The vessel is maintained under reflux for about 1 to about 10 hours, preferably 2 to 8 hours and most preferably 3 to 5 hours.

After boiling under reflux the reaction mixture is cooled down and the reaction product is separated from the solvents. The separation can be carried out by adding an amount of water to the reaction mixture which is sufficient to precipitate the benzoxazine compound A. The benzoxazine compound A is then isolated by filtration and can be dried at temperatures from 10°C to 70°C under reduced pressure.

Following the above procedure, the yield of the benzoxazine compound A ranges generally from 50 to 99 % of the theoretical yield.

To obtain the benzoxazine compound A of the present invention it is preferred that the stoichiometric ratio of primary amino groups in the primary amine of formula (VII) to phenolic hydroxyl groups in the phenolic compound is in the range of 0.5 to 2.0, more preferably 0.6 to 1.4, even more preferable 0.8 to 1.2 and most preferable about 1 or about 1.2.

To form one benzoxazine ring, one primary amino group, one phenolic hydroxyl group and two formaldehyde molecules are necessary. However it is preferred to use the formaldehyde in excess, the excess preferably being 10 % by mol, if a higher degree of ring-closed structures in the benzoxazine compound A of the present invention is desired.

Although all the formaldehyde may be provided as formalin, this is an undesirable method because formalin is expensive and it introduces an unnecessary amount of water into the system which must be removed later. However employing formalin in addition to paraformaldehyde in preparing the benzoxazine compound A of the present invention is advantageous. Paraformaldehyde is preferred as it is significantly less expensive than formalin. Employing formalin in combination with the paraformaldehyde provides enough water and methanol to dissolve the paraformaldehyde. Alternatively, just water may be used. Formalin is also advantageous in that it mitigates the exotherm reaction that occurs at about 80 °C to 85 °C. A violent exotherm reaction occurs because as water is generated more paraformaldehyde can dissolve, thus rapidly accelerating the reaction rate. Thus it is advantageous to employ a paraformaldehyde/formalin ratio of at least 1:1, based on the dry weight of the formaldehyde, and preferably of about 8:1 and more. However taking into account the abovementioned drawback, formaldehyde can be employed in water-free form such as paraformalehyde, trioxane or polyoxymethylene only, paraformaldeyde being most preferred.

As noted above, a decisive advantage of the present invention may be seen in the fact that it provides a benzoxazine compound A, which can be cured in an environmentally friendly process at temperatures of less than 200°C in short time periods without using additional curatives/catalysts which could negatively contribute to the final cured reaction product and its practical properties.

As noted above, it is another object of the invention to provide an adhesive, sealant or coating, comprising the benzoxazine compound A of the present invention and a cured reaction product of the benzoxazine compound A, in particular a cured reaction product containing bundles or layers of fibers.

Preferably, the inventive benzoxazine compound A is cured at temperatures from 80°C to 200°C, preferably from 90°C to 180°C, and more preferably from 100°C to 160°C in less than 6 hours, preferably in less than 5 hours, and more preferably in less than 3 hours and/or at pressures between 1 to 100 atm, preferably between 1 to 5 atm, and more preferably under atmospheric pressure.

Another object of the present invention is a polymerizable composition, comprising
a) at least one benzoxazine compound A of the present invention; and
b) at least one benzoxazine compound B, which is different from benzoxazine compound A.

The term "which is different from" as used herein refers to the chemical structure of benzoxazine compound A and benzoxazine compound B. A benzoxazine compound B which is different from the benzoxazine compound A of the present invention is not represented by formula (I).

In a particular preferred embodiment of the present invention the at least one benzoxazine compound B, which is different from benzoxazine compound A, comprises at least one benzoxazine compound of formula (V) or formula (VI), 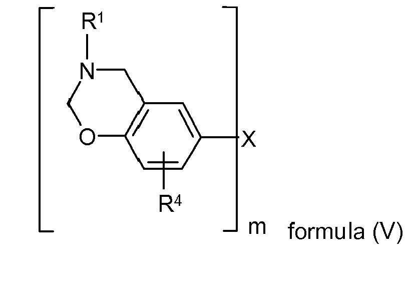 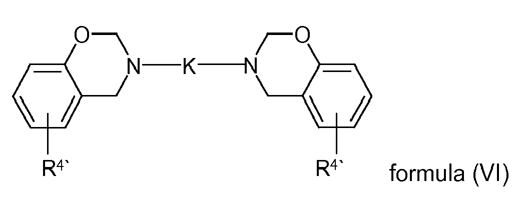 wherein m is an integer from 1 to 4, X is selected from the group consisting of a direct bond (when m is 2), hydrogen (when m is 1), alkyl (when m is 1), alkylene (when m is 2 to 4), carbonyl (when m is 2), oxygen (when m is 2), thiol (when m is 1), sulfur (when m is 2), sulfoxide (when m is 2), and sulfone (when m is 2), R¹ is selected from hydrogen, alkyl, alkenyl and aryl, and R¹ does not comprise any carboxyl group or a corresponding salt thereof, K is selected from the group consisting of biphenyl, diphenyl methane, diphenyl isopropane, diphenyl sulfide, diphenyl sulfoxide, diphenyl sulfone, and diphenyl ketone, and R⁴ is selected from hydrogen, halogen, alkyl, alkenyl or R⁴ is a divalent residue creating a naphthoxazine residue out of the benzoxazine structure and R^{4'} is defined as R⁴

In a preferred embodiment of the present invention R⁴ and/or R^{4'} is/are hydrogen.

More specifically, within formula (VI) the at least one benzoxazine compound B of the present invention may be embraced by the following structure (B-IX), 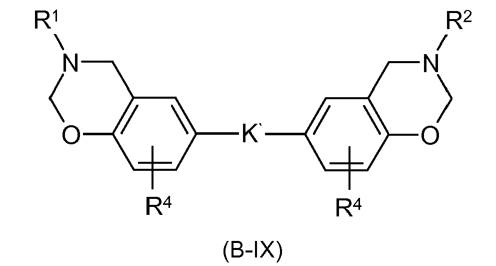 where K' is selected from a direct bond, CH₂, C(CH₃)₂, C=O, O, S, S=O and O=S=O, R¹ and R² are the same or different, and R¹ is defined as above and R² is defined as R¹, and R⁴ are the same or different and defined as above.

Representative benzoxazines within structure (B-IX) include: 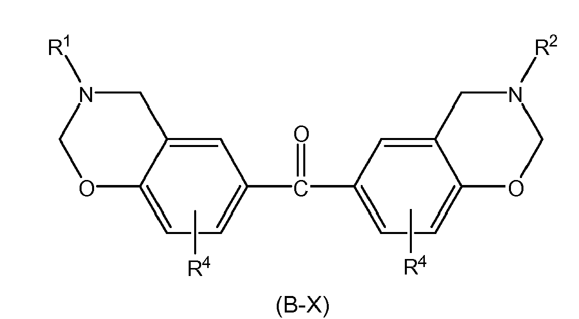 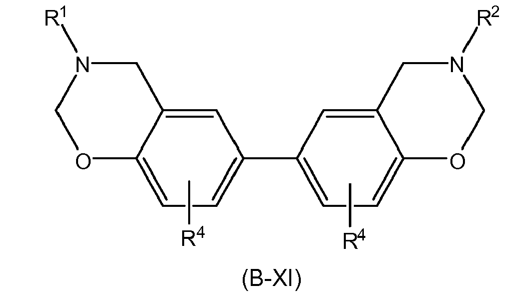 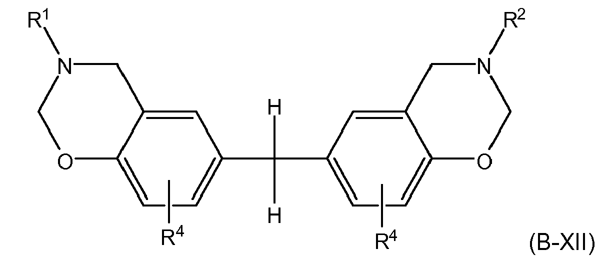 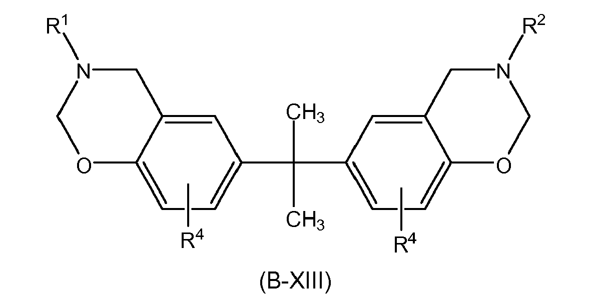 wherein R¹, R² and R⁴ are as defined above.

Though not embraced by formula (V) and/or formula (VI) additional benzoxazine compounds B of the present invention are within the following structures: 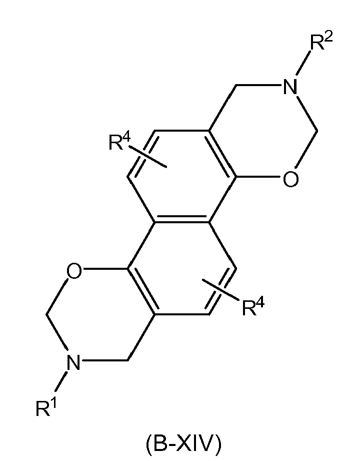 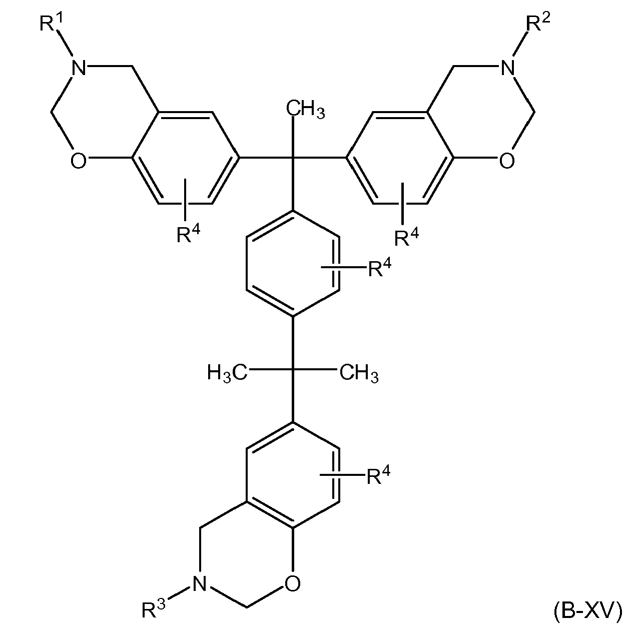 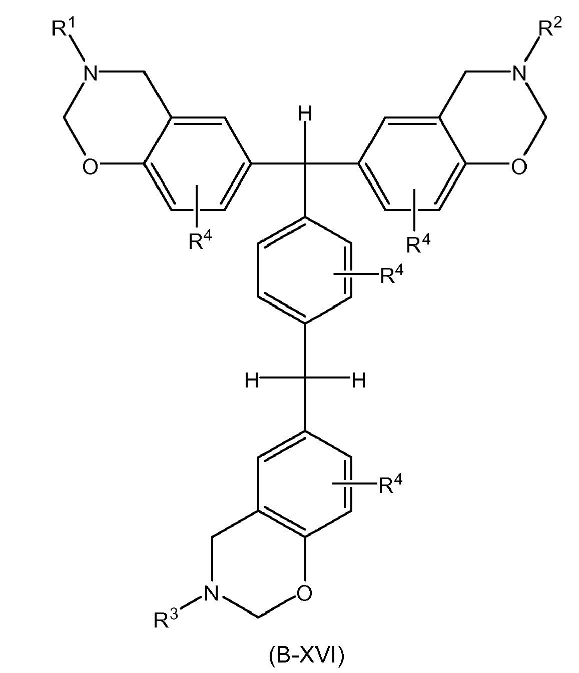 wherein R¹, R² and R⁴ are as defined above, and R³ is defined as R¹.

Specific examples of the above generically described benzoxazines include:

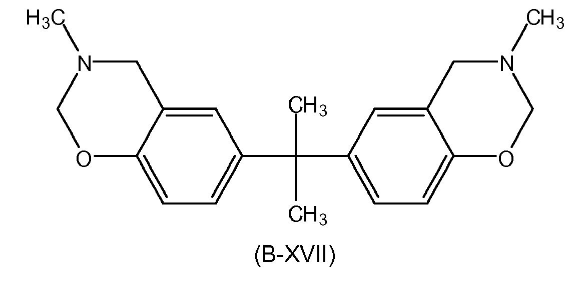

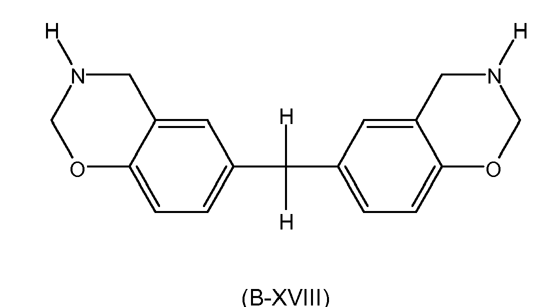

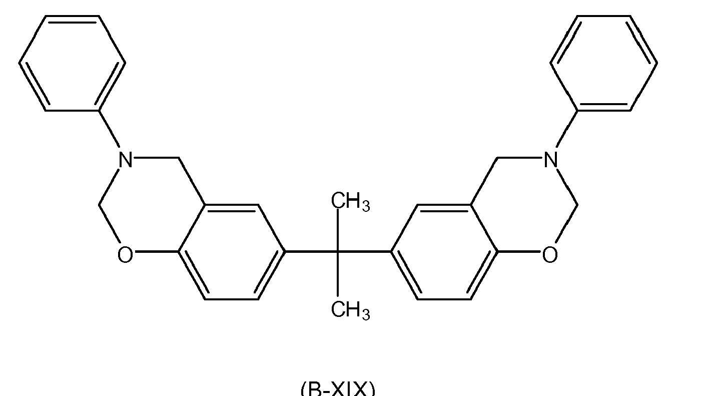

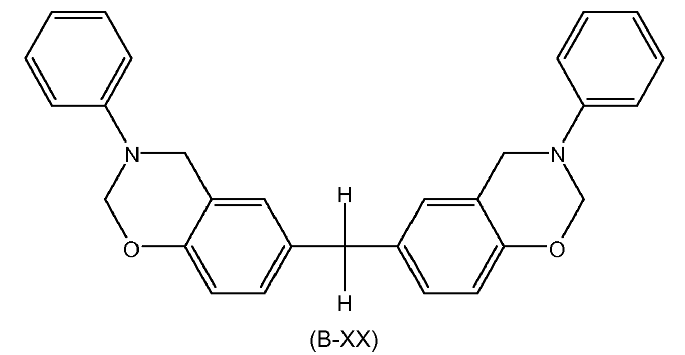

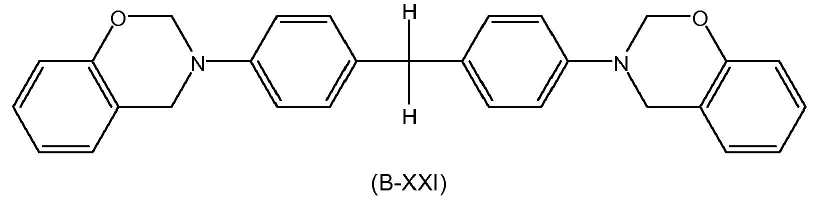

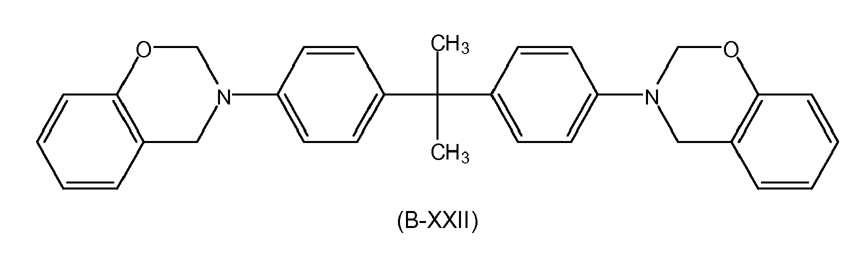

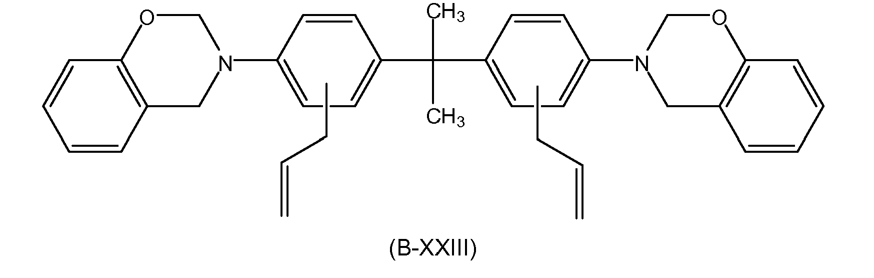

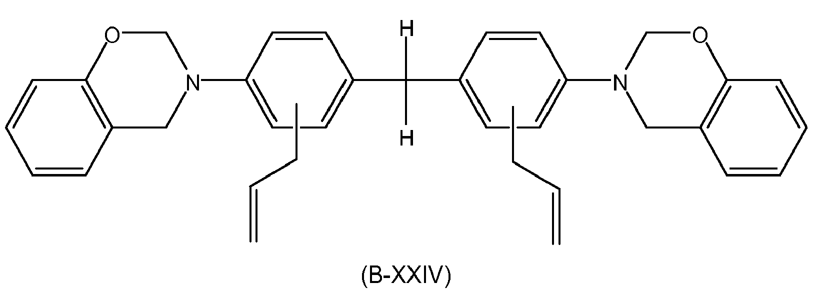

The at least one benzoxazine compound B according to formula (V) and/or formula (VI) may include the combination of multifunctional benzoxazines and monofunctional benzoxazines, or may be the combination of one or more multifunctional benzoxazines or one or more monofunctional benzoxazines.

Examples of monofunctional benzoxazines may be embraced by the following structure (B-XXV): 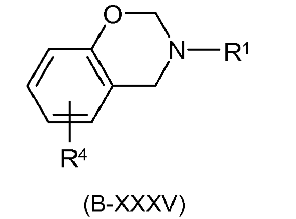 wherein R¹ and R⁴ are defined as above.

For instance, monofunctional benzoxazines may be embraced by general structure (B-XXVI), 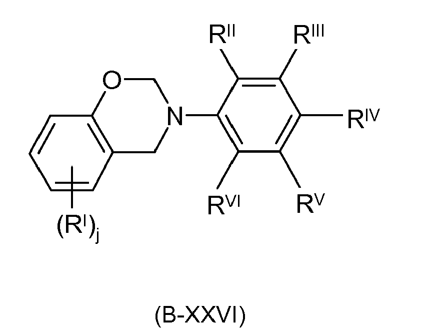 where in this case R^{I} is selected from alkyl, alkenyl, each of which being optionally substituted or interrupted by one or more O, N, S, C=O, COO, and NHC=O, and aryl; j is an integer from 0 to 4; and each R^{II}, R^{III}, R^{IV}, R^{V} and R^{VI} does not comprise any carboxyl group or a corresponding salt thereof, and R^{II}, R^{III}, R^{IV}, R^{V} and R^{VI} are independently selected from hydrogen, alkyl, alkenyl, each of which being optionally substituted or interrupted by one or more S, C=O, and aryl.

Specific examples of such a monofunctional benzoxazine are: 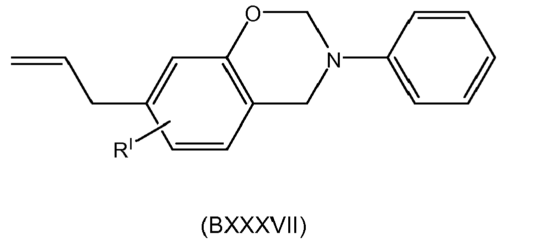 wherein R^{I} is as defined above; or

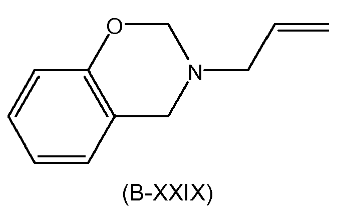

In a particularly preferred embodiment of the present invention the at least one benzoxazine compound B, which is different from benzoxazine compound A, is an "aliphatic benzoxazine", i.e. a benzoxazine having an aliphatic residue bound to the nitrogen atoms of the benzoxazine residue, such as the compound of formula (B-XXIX) above. However in another preferred embodiment it can be desirable to use "aromatic benzoxazines", i.e. benzoxazines having an aromatic residue bound to the nitrogen atoms of the benzoxazine residues such as the compounds of formulas (B-XIX) or (B-XX). In some other preferred embodiments mixtures of the before-mentioned benzoxazines are advantageously employed.

Additionally, in some other preferred embodiments of the present invention the polymerizable composition of the present invention may comprise a mixture of at least one benzoxazine according to formula (V) and at least one benzoxazine according to formula (VI).

Benzoxazines are presently available commercially from several sources, including Huntsman Advanced Materials; Georgia-Pacific Resins, Inc.; and Shikoku Chemicals Corporation, Chiba, Japan.

If desired, however, instead of using commercially available sources, the benzoxazines of the present invention may typically be prepared by reacting a phenolic compound, such as a bisphenol A, bisphenol F, bisphenol S or thiodiphenol, with an aldehyde and an alkyl or aryl amine. U.S. Patent No. 5,543,516, hereby expressly incorporated herein by reference, describes a method of forming benzoxazines, where the reaction time can vary from a few minutes to a few hours, depending on reactant concentration, reactivity and temperature. See generally U.S. Patent Nos. 4,607,091 (Schreiber), 5,021,484 (Schreiber), 5,200,452 (Schreiber) and 5,443,911 (Schreiber).

The polymerizable composition can comprise at least one benzoxazine compound A of the present invention and at least one benzoxazine compound B, which is different from benzoxazine compound A in a molar ratio of from 99.9:0.1 to 0.1:99.9, preferably from 40:60 to 1:99, and more preferably from 50:50 to 2:98 Particularly preferred molar ratios of benzoxazine compound A to benzoxazine compound B are 2:98, 3:97, 4:96, 5:95, 6:94, 7:93, 8:92, 9:91, 10:90, 15:85, 20:80, 25:75, 30:70, 35:65, 40:60 and 45:65.

In one embodiment the polymerizable composition comprises at least one benzoxazine compound A of the present invention in an amount from about 5 to about 95 percent by weight, preferably from about 20 to about 80 percent by weight and more preferably from about 40 to 60 percent by weight, based on the total amount of the polymerizable composition.

In another embodiment, the polymerizable composition comprises at least one benzoxazine compound B, which is different from benzoxazine compound A, in an amount from about 5 to about 95 percent by weight, preferably from about 20 to about 80 percent by weight and more preferably from about 40 to 60 percent by weight, based on the total amount of the polymerizable composition.

Preferably, the total amount of all benzoxazine compounds in the polymerizable composition is in the range from about 10 to about 100 percent by weight, preferably from about 20 to about 99 percent by weight, and more preferably from about 50 to 95 percent by weight, based on the total amount of the polymerizable composition.

The polymerizable composition of the present invention may further comprises one or more epoxy resins, i.e. epoxy-containing compounds even though the addition of epoxy resins is not necessary. Preferably the amount of epoxy resins employed does not exceed 60 wt.-%, more preferably 40 wt.-% and most preferably 20 wt.-%.

The term "epoxy resin", as used in the present invention, refers to any organic compound having at least two functional groups of oxirane type which can be polymerized by ring opening. The term "epoxy resin components" preferably denotes any conventional epoxy resin which is liquid at room temperature (23°C) or at a higher temperature. These epoxy resins can be monomeric or polymeric, on the one hand, aliphatic, cycloaliphatic, heterocyclic or aromatic, on the other hand.

The epoxy resins used in the present invention may include multifunctional epoxy- containing compounds, such as C₁-C₂₈ alkyl-, poly-phenol glycidyl ethers; polyglycidyl ethers of pyrocatechol, resorcinol, hydroquinone, 4,4'-dihydroxydiphenyl methane (or bisphenol F, such as RE-303-S or RE-404-S available commercially from Nippon Kayuku, Japan), 4,4'-dihydroxy-3,3'-dimethyldiphenyl methane, 4,4'-dihydroxydiphenyl dimethyl methane (or bisphenol A), 4,4'-dihydroxydiphenyl methyl methane, 4,4'-dihydroxydiphenyl cyclohexane, 4,4'-dihydroxy-3,3'-dimethyldiphenyl propane, 4,4'-dihydroxydiphenyl sulfone, and tris(4-hydroxyphenyl) methane; polyglycidyl ethers of transition metal complexes; chlorination and bromination products of the above-mentioned diphenols; polyglycidyl ethers of novolacs; polyglycidyl ethers of diphenols obtained by esterifying ethers of diphenols obtained by esterifying salts of an aromatic hydrocarboxylic acid with a dihaloalkane or dihalogen dialkyl ether; polyglycidyl ethers of polyphenols obtained by condensing phenols and long-chain halogen paraffins containing at least two halogen atoms; phenol novolac epoxy; cresol novolac epoxy; and combinations thereof.

Among the commercially available epoxy resins suitable for use in the present invention are polyglycidyl derivatives of phenolic compounds, such as those available under the tradenames EPON 825, EPON 826, EPON 828, EPON 1001, EPON 1007 and EPON 1009, cycloaliphatic epoxy-containing compounds such as Araldite CY179 from Huntsman or waterborne dispersions under the tradenames EPI-REZ 3510, EPI-REZ 3515, EPI-REZ 3520, EPI-REZ 3522, EPI-REZ 3540 or EPI-REZ 3546 from Hexion; DER 331, DER 332, DER 383, DER 354, and DER 542 from Dow Chemical Co.; GY285 from Huntsman, Inc.; and BREN-S from Nippon Kayaku, Japan. Other suitable epoxy-containing compounds include polyepoxides prepared from polyols and the like and polyglycidyl derivatives of phenol-formaldehyde novolacs, the latter of which are available commercially under the tradenames DEN 431, DEN 438, and DEN 439 from Dow Chemical Company and a waterborne dispersion ARALDITE PZ 323 from Huntsman.

Cresol analogs are also available commercially such as ECN 1273, ECN 1280, ECN 1285, and ECN 1299 or waterborne dispersions ARALDITE ECN 1400 from Huntsman, Inc. SU-8 and EPI-REZ 5003 are bisphenol A-type epoxy novolacs available from Hexion.

Of course, combinations of the different epoxy resins are also desirable for use herein.

One or more epoxy resins can be used in the polymerizable composition in an amount of preferably 0 to 60 percent by weight, more preferably 5 to 50 percent by weight, and most preferably 6 to 20 percent by weight, based on the total amount of the polymerizable composition of the present invention.

The polymerizable composition of the present invention can further comprise additional curable ingredients other than benzoxazine-based ingredients and epoxy-based ingredients such as phenol resins, maleinimide resins, oxazolines, isocyanates and/or mixtures or combinations thereof.

If desired, reactive diluents, for example styrene oxide (epoxide derived from styrene), butyl glycidyl ether, 2,2,4-trimethylpentyl glycidyl ether, phenyl glycidyl ether, cresyl glycidyl ether or glycidyl esters of synthetic, highly branched, mainly tertiary, aliphatic monocarboxylic acids, oxazoline group containing compounds may be added to the polymerizable composition to reduce its viscosity.

Other additives which the inventive polymerizable composition can include are tougheners, plasticizers, extenders, microspheres, fillers and reinforcing agents, for example coal tar, bitumen, textile fibers, glass fibers, asbestos fibers, boron fibers, carbon fibers, mineral silicates, mica, powdered quartz, hydrated aluminum oxide, bentonite, wollastonite, kaolin, silica, aerogel or metal powders, for example aluminium powder or iron powder, and also pigments and dyes, such as carbon black, oxide colors and titanium dioxide, fire-retarding agents, thixotropic agents, flow control agents, such as silicones, waxes and stearates, which can, in part, also be used as mold release agents, adhesion promoters, antioxidants and light stabilizers, the particle size and distribution of many of which may be controlled to vary the physical properties and performance of the inventive polymerizable composition.

When used, fillers are used in an amount sufficient to provide the desired rheological properties. Fillers may be used in an amount up to about 50 percent by weight, such as about 5 to about 32 percent by weight, for instance about 10 to about 25 percent by weight.

The fillers may be inorganic ones, such as silicas. For instance, the silica filler may be a silica nanoparticle.

In a typical embodiment of the present invention the polymerizable composition comprises/consists of, based on the total amount of the composition:
a) from 5 to 95 percent by weight, more typically from 20 to 80 percent by weight, suitably from 40 to 60 percent by weight of at least one benzoxazine compound A of the present invention;
b) from 5 to 95 percent by weight, more typically from 20 to 80 percent by weight, suitably from 40 to 60 percent by weight of at least one benzoxazine compound B;
c) from 0 to 60 percent by weight, more typically from 5 to 50 percent by weight, suitably from 10 to 30 percent by weight, for example from 15 to 25 percent by weight of at least one epoxy resin; and
d) from 0 to 30 percent by weight, more typically from 2 to 20 percent by weight, suitably from 5 to 15 percent by weight, for example from 6 to 12 percent by weight of one or more additives.

In one embodiment of the present invention the inventive polymerizable composition is cured at temperatures from 50°C to 200°C, preferably from 80°C to 180°C, more preferably from 100°C to 160°C and/or at pressures between 1 to 100 atm, preferably between 1 to 5 atm, and more preferably under atmospheric pressure.

The polymerizable composition of the present invention can also be supplemented with additional curatives without losing their advantages properties in case the use of additional curatives is desired for specific applications.

In this regard Lewis acids, and other known curatives, such as metal halides; organometallic derivatives; metallophorphyrin compounds such as aluminum phthalocyanine chloride; anhydrides, methyl tosylate, methyl triflate, and triflic acid; and oxyhalides can be added to the polymerizable composition of the present invention.

However taking into account that the aforementioned curatives could cause the formation of volatile, toxic and corrosive impurities, polymerizable compositions are preferred that do not comprise the aforementioned additional curatives.

As noted, the polymerizable compositions of the present invention are in particular suitable as coatings, adhesives, sealants and matrices for the preparation of reinforced material such as prepregs and towpregs and/or can be used in injection molding or extrusion.

In this regard, it is another object of the invention to provide an adhesive, sealant or coating comprising the polymerizable composition of the present invention.

The invention also provides a cured reaction product of the polymerizable composition, in particular cured reaction products containing bundles or layers of fibers infused with the polymerizable composition, and a method of preparing such material.

In this regard, the invention relates to processes for producing a prepreg or a towpregs. One such process includes the steps of (a) providing a layer or bundle of fibers; (b) providing a polymerizable composition of the present invention; (c) joining said polymerizable composition and the layer or bundle of fibers to form a prepreg or a towpregs assembly; and (d) optionally removing excess polymerizable composition from the prepreg or towpreg assembly, and exposing the resulting prepreg or towpreg assembly to elevated temperature and pressure conditions sufficient to infuse the layer or bundle of fibers with the polymerizable composition to form a prepreg or a towpregs assembly as the cured reaction product.

Another such process for producing a prepreg or towpreg, includes the steps of (a) providing a layer or bundle of fibers; (b) providing a polymerizable composition of the present invention in liquid form; (c) passing the layer or bundle of fibers through said polymerizable composition to infuse the layer or bundle of fibers with said polymerizable composition; and (d) removing excess of said polymerizable composition from the prepreg or towpreg assembly, and exposing the resulting prepreg or towpreg assembly to elevated temperature and pressure conditions sufficient to infuse the layer or bundle of fibers with the polymerizable composition to form a prepreg or a towpregs assembly as the cured reaction product.

Generally, the fiber layer or bundle may be constructed from unidirectional fibers, woven fibers, chopped fibers, non-woven fibers or long, discontinuous fibers.

The fiber chosen may be selected from carbon, glass, aramid, boron, polyalkylene, quartz, polybenzimidazole, polyetheretherketone, polyphenylene sulfide, poly p-phenylene benzobisoaxazole, silicon carbide, phenolformaldehyde, phthalate and napthenoate.

The carbon may be selected from polyacrylonitrile, pitch and acrylic, and the glass is selected from S glass, S2 glass, E glass, R glass, A glass, AR glass, C glass, D glass, ECR glass, glass filament, staple glass, T glass and zirconium oxide glass.

The inventive polymerizable composition (and prepregs and towpregs prepared therefrom) is particularly useful in the manufacture and assembly of composite parts for aerospace and industrial end uses, bonding of composite and metal parts, core and core-fill for sandwich structures and composite surfacing.

The inventive polymerizable composition is also useful as a coating, sealant or adhesive for the electronics industry. Suitable substrates on which the polymerizable compositions of the present invention are applied are metals such as steel, aluminum, titanium, magnesium, brass, stainless steel, galvanized steel, like HDG-steel and EG-steel; silicates such as glass and quartz; metal oxides; concrete; wood; electronic chip material, for instance semiconductor chip material; or polymers such as polyimide films and polycarbonate.

The invention also relates to the use of the at least one benzoxazine compound A of the present invention as a curative/catalyst for curable compositions, comprising at least one benzoxazine compound, which is different from benzoxazine compound A.

The inventors of the present invention surprisingly found that by adding at least one benzoxazine compound A to a curable composition, comprising at least one benzoxazine compound, which is different from benzoxazine compound A, the polymerization rate of said curable composition can be increased at temperatures up to 200°C.

In this regard the invention relates to a method to increase the polymerization rate of a curable composition at temperatures up to 200°C, steps of which comprise:
a) adding at least one benzoxazine compound A of the present invention to a curable composition;
b) subjecting the curable composition to conditions appropriate to cure the curable composition,
wherein the curable composition comprises at least one benzoxazine compound, which is different from benzoxazine compound A.

The term "polymerization rate" as used herein means an average value of the amounts of a change in polymerization conversion per every unit hour (%/hour) obtained in the first 4 hours after starting the polymerization. The polymerization rate can easily be determined by a man skilled in the art using known techniques, such as GC-analysis, NMR- or IR spectroscopy.

In preferred embodiments of the present invention the polymerization rate is determined at temperatures from 80°C to 200°C, preferably from 90°C to 180°C, and more preferably from 100°C to 160°C and/or at pressures between 1 to 100 atm, preferably between 1 to 5 atm, and more preferably under atmospheric pressure.

The term curable composition, as used in the present invention, refers to a composition which comprises/consists of at least one benzoxazine compound, which is different from benzoxazine compound A, such as benzoxazine compound B.

In a particular preferred embodiment of the present invention the curable composition comprises at least one benzoxazine compound B in an amount from about 5 to about 100 percent by weight, preferably from about 20 to about 99 percent by weight and more preferably from about 40 to about 95 percent by weight, based on the total amount of the curable composition of the present invention.

Preferably, the curable composition of the present invention is cured at temperatures from 50°C to 200°C, preferably from 80°C to 180°C, and more preferably from 100°C to 160°C and/or at pressures between 1 to 100 atm, preferably between 1 to 5 atm, and more preferably under atmospheric pressure.

A decisive advantage of the present invention may be seen in the fact that the benzoxazine compounds A of the present invention significantly increase the polymerization rate of the aforementioned curable compositions at temperatures up to 200°C without causing the formation of undesired volatile, toxic and corrosive impurities.

The invention is further illustrated by the following examples.

### EXAMPLES

### A. Synthesis of benzoxazine compound 1a

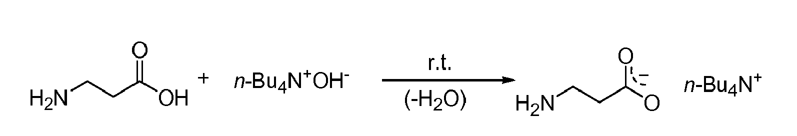

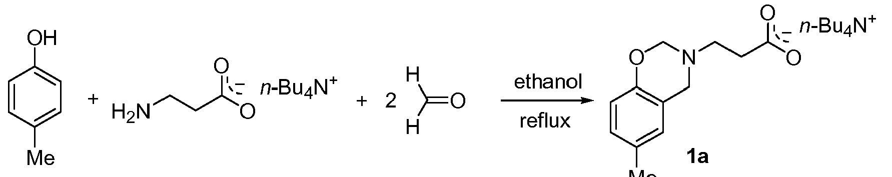

To a solution of beta-alanine (2.2 g, 25 mmol) in H₂O (50 mL), a tetrabutylammonium hydroxide solution in methanol (10 mL, 20 mmol) was added at 0 °C dropwise for 10 minutes and stirred at 22°C for 15 h. The mixture was poured into acetonitrile, and the resulting precipitate of excess glycine was removed by filtration. The filtrate was concentrated in vacuum at 70°C to obtain the corresponding tetrabutylammonium salt of beta-alanine (beta-Ala-TBA) as a liquid (5.0 g, yield 80 %). The obtained beta-Ala-TBA (3.3 g, 10 mmol), *p*-cresol (1.3 g, 12 mmol), and formaldehyde (1.6 mL, 20 mmol) were dissolved in ethanol (60 mL), and the solution was heated under refluxing for 8 h. The resulting mixture was diluted with water and filtered. Water was removed under reduced pressure, and the residue was fractionated by column chromatography to give 1 a (6.6 g, yield 80%).

### B. Synthesis of benzoxazine compound 1 b

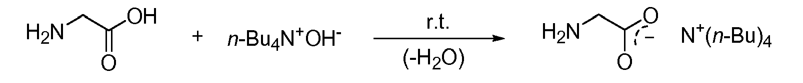

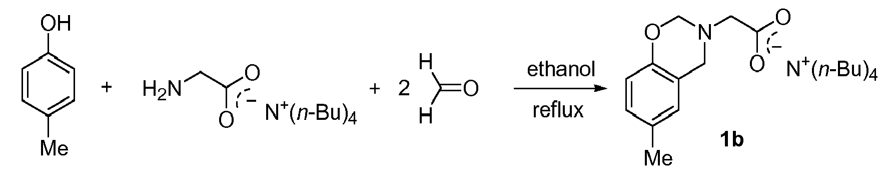

To a solution of glycine (1.9 g, 25 mmol) in H₂O (50 mL), a tetrabutylammonium hydroxide solution in methanol (10 mL, 20 mmol) was added at 0 °C dropwise for 10 minutes and stirred 22°C for 15 h. The mixture was poured into acetonitrile, and the resulting precipitate of excess glycine was removed by filtration. The filtrate was concentrated in vacuum at 70 °C to obtain the corresponding tetrabutylammonium salt of glycine (Gly-TBA) as a liquid (5.0 g, yield 80 %). The obtained Gly-TBA (3.2 g, 10 mmol), *p*-cresol (1.3 g, 12 mmol), and formaldehyde (1.6 mL, 20 mmol) were dissolved in ethanol (60 mL), and the solution was heated under refluxing for 3 h. The resulting mixture was diluted with water, filtered, and concentrated in vacuum at 30 °C to obtain 1 b as a liquid (3.0 g, yield 67 %).

### C. Example 1-1: Polymerization of 1a at 150°C

An amount of 3.1 g 1a (6.7 mmol) was divided into 6 portions (500 mg each) and each portion was placed in a test tube. To the resulting 6 test tubes Argon inlets were attached and the test tubes were heated in an oil bath at 150 °C. From time to time, the test tubes were taken away from the oil bath one-by-one, and each mixtures was analyzed by ¹H-NMR to determine the conversion of 1a. The results are given in Table 1-1.

**Table 1-1**

| Time/h | 1 | 2 | 3 | 4 | 5 | 6 |
|---|---|---|---|---|---|---|
| Conversion/% | 90 | 97 | 100 | 100 | 100 | 100 |

### D. Example 1-2: Polymerization of 1b at 150°C

An amount of 3.0 g 1 b (6.7 mmol) was divided into 6 portions (500 mg each) and each portion was placed in a test tube. To the resulting 6 test tubes Argon inlets were attached and the test tubes were heated in an oil bath at 150 °C. From time to time, the test tubes were taken away from the oil bath one-by-one, and each mixture was analyzed by ¹H-NMR to determine the conversion of 1a. The results are given in Table 1-2.

**Table 1-2**

| Time/h | 1 | 2 |
|---|---|---|
| Conversion/% | 100 | 100 |

### E. Comparative Example 1-1: Polymerization of N-(2-hydroxyethyl)benzoxazine 1 c at 150°C

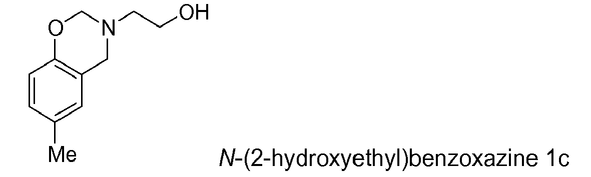

*N*-(2-hydroxyethyl)benzoxazine 1c (1.29 g. 6.7 mmol) was divided into 6 portions (500 mg each) and each portion was placed in a test tube. To the resulting 6 test tubes Argon inlets were attached and the test tubes were heated in an oil bath at 150 °C. From time to time, the test tubes were taken away from the oil bath one-by-one, and each mixture was analyzed by ¹H-NMR to determine the conversion of 1a. The results are given in Table 1-3.

**Table 1-3**

| Time/h | 1 | 2 | 3 | 4 | 5 | 6 |
|---|---|---|---|---|---|---|
| Conversion/% | 89 | 92 | 94 | 95 | 97 | 98 |

### F. Comparative Example 1-2: Polymerization of N-methylbenzoxazine 1d at 150°C

| | |
|---|---|
| 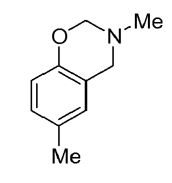 | *N*-methylbenzoxazine 1d |

*N*-methylbenzoxazine 1d (1.09 g. 6.7 mmol) was divided into 6 portions (500 mg each) and each portion was placed in a test tube. To the resulting 6 test tubes Argon inlets were attached and the test tubes were heated in an oil bath at 150 °C. From time to time, the test tubes were taken away from the oil bath one-by-one, and each mixture was analyzed by ¹H-NMR to determine the conversion of 1a. The results are given in Table 1-4.

**Table 1-4**

| Time/h | 1 | 2 | 4 |
|---|---|---|---|
| Conversion/% | 69 | 85 | 88 |

### G. Example 2-1: Polymerization of 1 a at 120°C

The time-conversion relationships for 1 a were determined at 120°C in accordance with the procedure described in Example 1-1. The results are given in Table 2-1.

**Table 2-1**

| Time/h | 1 | 2 | 3 | 4 | 5 | 6 |
|---|---|---|---|---|---|---|
| Conversion/% | 50 | 70 | 75 | 80 | 82 | 90 |

### H. Example 2-2: Polymerization of 1 b at 120°C

The time-conversion relationships for 1 b were determined in accordance with the procedure described in Example 1-1 at 120°C. The results are given in Table 2-2.

**Table 2-2**

| Time/h | 1 | 2 | 3 | 4 | 5 | 6 |
|---|---|---|---|---|---|---|
| Conversion/% | 77 | 88 | 95 | 97 | 98 | 100 |

### I. Comparative Example 2-1: Polymerization of N-(2-hydroxyethyl)benzoxazine 1c at 120°C The time-conversion relationships for N-(2-hydroxyethyl)benzoxazine 1c were determined at 120°C in accordance with the procedure described in Example 1-1. The results are given in Table 2-3.

**Table 2-3**

| Time/h | 1 | 2 | 3 | 4 | 5 | 6 |
|---|---|---|---|---|---|---|
| Conversion/% | 44 | 74 | 88 | 88 | 90 | 93 |

### J. Comparative Example 2-2: Polymerization of N-Methylbenzoxazine 1d at 120°C

The time-conversion relationships for *N*-methylbenzoxazine 1 d were determined at 120°C in accordance with the procedure described in Example 1-1. The results are given in Table 2-4.

**Table 2-4**

| Time/h | 1 | 2 | 3 | 4 | 5 | 6 |
|---|---|---|---|---|---|---|
| Conversion/% | 13 | 17 | 19 | 20 | 24 | 32 |

### K. Example 3-1: Polymerization of 1 a at 100°C

The time-conversion relationships for 1 a were determined at 100°C in accordance with the procedure described in Example 1-1. The results are given in Table 3-1.

**Table 3-1**

| Time/h | 1 | 2 | 3 | 4 | 5 | 6 |
|---|---|---|---|---|---|---|
| Conversion/% | 20 | 37 | 38 | 40 | 45 | 50 |

### L. Example 3-2: Polymerization of 1 b at 100°C

The time-conversion relationships for 1 b were determined at 100°C in accordance with the procedure described in Example 1-1. The results are given in Table 3-2.

**Table 3-2**

| Time/h | 1 | 2 | 3 | 4 | 5 | 6 |
|---|---|---|---|---|---|---|
| Conversion/% | 40 | 63 | 63 | 65 | 70 | 78 |

### M. Comparative Example 3-1: Polymerization of N-(2-hydroxyethyl)benzoxazine 1c at 100°C The time-conversion relationships for N-(2-hydroxyethyl)benzoxazine 1c were determined at 100°C in accordance with the procedure described in Example 1-1. The results are given in Table 3-3.

**Table 3-3**

| Time/h | 1 | 2 | 3 | 4 | 5 | 6 |
|---|---|---|---|---|---|---|
| Conversion/% | 15 | 25 | 30 | 32 | 40 | 45 |

### N. Comparative Example 3-2: Polymerization of N-Methylbenzoxazine 1d at 100°C

The time-conversion relationships for *N*-methylbenzoxazine 1 d were determined at 100°C in accordance with the procedure described in Example 1-1. The results are given in Table 3-4.

**Table 3-4**

| Time/h | 1 | 2 | 3 | 4 | 5 | 6 |
|---|---|---|---|---|---|---|
| Conversion/% | 0 | 0 | 0 | 0 | 0 | 0 |

The examples show that the polymerization rate of benzoxazine compound A significantly higher than the polymerization rate of comparable benzoxazines, which do not comprise at least one carboxyl or a corresponding salt thereof.

## Claims

1. A benzoxazine compound A of formula (I), 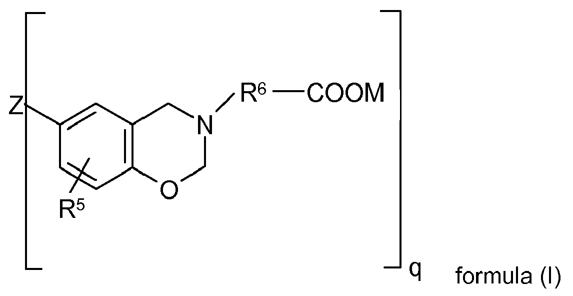 wherein q is an integer from 1 to 4, M is hydrogen, a monovalent cation, or an equivalent of a polyvalent cation, Z is selected from the group consisting of a direct bond (when q is 2), hydrogen (when q is 1), alkyl (when q is 1), alkylene (when q is 2 to 4), carbonyl (when q is 2), oxygen (when q is 2), thiol (when q is 1), sulfur (when q is 2), sulfoxide (when q is 2), and sulfone (when q is 2), R⁶ is a linear or branched divalent alkylene group, comprising 1 to 15 carbon atoms, which may be interrupted by one or more heteroatom(s), selected from oxygen, nitrogen and sulfur, and R⁵ is selected from hydrogen, halogen, alkyl, alkenyl or R⁵ is a divalent residue creating a naphthoxazine residue out of the benzoxazine structure.

2. The benzoxazine compound A of Claim 1, wherein R⁶ is a linear divalent alkylene group comprising 1 to 4 carbon atoms.

3. The benzoxazine compound A of Claim 1, wherein R⁶ is a linear divalent alkylene group, which is interrupted by at least one oxygen atom.

4. The benzoxazine compound A of any one of Claims 1 to 3, wherein M is a monovalent cation, which comprises at least one heteroatom selected from nitrogen, phosphorous or sulfur.

5. The benzoxazine compound A of Claim 4, **characterized in that** M is a monovalent cation selected from
- quaternary ammonium cations of general formula (II)
[NR^{a}R^{b}R^{c}R^{d}]⁺ formula (II)
- phosphonium cations of general formula (III), or
[PR^{a}R^{b}R^{c}R^{d}]⁺ formula (III)
- sulfonium cations of general formula (IV),
[SR^{a}R^{b}R^{c}]⁺ formula (IV)
wherein the residues R^{a}, R^{b}, R^{c}, and R^{d} (if present) are independently selected from C₁-C₄₀ alkyl groups, C₃-C₄₀ cycloalkyl groups, C₃₋₄₀ alkenyl groups, C₃₋₄₀ alkynyl groups, C₇-C₄₀ aralkyl groups, C₆-C₄₀ aryl groups or C₇-C₄₀ aralkyl groups.

6. Polymerizable composition, comprising
a) at least one benzoxazine compound A of any one of Claims 1 to 5;
b) at least one benzoxazine compound B, which is different from benzoxazine compound A.

7. Polymerizable composition of Claim 6, wherein the benzoxazine compound B comprises at least one benzoxazine compound of general formula (V) or general formula (VI), 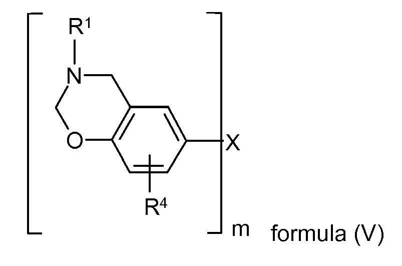 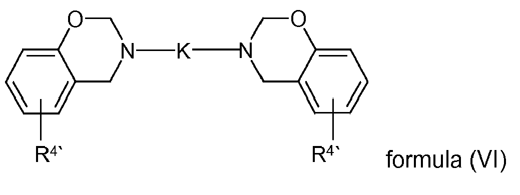 wherein m is an integer from 1 to 4, X is selected from the group consisting of a direct bond (when m is 2), hydrogen (when m is 1), alkyl (when m is 1), alkylene (when m is 2 to 4), carbonyl (when m is 2), oxygen (when m is 2), thiol (when m is 1), sulfur (when m is 2), sulfoxide (when m is 2), and sulfone (when m is 2), R¹ is selected from hydrogen, alkyl, alkenyl and aryl and R¹ does not comprise any carboxyl group,
K is selected from the group consisting of biphenyl, diphenyl methane, diphenyl isopropane, diphenyl sulfide, diphenyl sulfoxide, diphenyl sulfone, and diphenyl ketone,
and R⁴ is selected from hydrogen, halogen, alkyl, alkenyl or R⁴ is a divalent residue creating a naphthoxazine residue out of the benzoxazine structure and R^{4'} is defined as R⁴

8. Polymerizable composition of Claim 6 and/or Claim 7, wherein said polymerizable composition comprises at least one benzoxazine compound A and at least one benzoxazine compound B in a molar ratio from 50 : 50 to 1 : 99.

9. Polymerizable composition of any one of Claims 6 to 8, wherein said polymerizable composition additionally comprises at least one epoxy resin.

10. Polymerizable composition of any one of Claims 6 to 9, wherein said polymerizable composition cures at temperatures from 50°C to 200°C.

11. A cured reaction product of the benzoxazine compound of any one of Claims 1 to 5 or of the polymerizable composition of any one of Claims 6 to 10.

12. An adhesive, sealant or coating composition, comprising the benzoxazine compound A of any one of Claims 1 to 5 or the polymerizable composition of any one of Claims 6 to 10.

13. A method of preparing a benzoxazine compound A of any one of Claims 1 to 5, comprising the steps of:
(a) providing a reaction mixture, comprising
i) at least one phenolic compound,
ii) at least one primary amine of formula (VII),
H₂N-R⁶-COOM formula (VII),
wherein M is hydrogen, a monovalent cation, or an equivalent of a polyvalent cation, R⁶ is a linear or branched divalent alkylene group, comprising 1 to 15 carbon atoms, which may be interrupted by one or more heteroatom(s), selected from oxygen, nitrogen and sulfur,
iii) formaldehyde or a reactant releasing formaldehyde, and
iv) at least one solvent;
(b) heating the reaction mixture under reflux,
(c) optionally removing water from the reaction mixture, and
(d) separating the benzoxazine compound A from the solvent.

14. A method to increase the polymerization rate of a curable composition at temperatures up to 200°C, steps of which comprise:
a) adding at least one benzoxazine compound A of any one of Claims 1 to 5 to a curable composition;
b) subjecting the curable composition to conditions appropriate to cure the curable composition,
wherein the curable composition comprises at least one benzoxazine compound, which is different from benzoxazine compound A.

15. Use of the at least one benzoxazine compound A of any one of Claims 1 to 5 as a curative for curable compositions, comprising at least one benzoxazine compound, which is different from benzoxazine compound A.
